(19) Europäisches Patentamt / European Patent Office / Office européen des brevets

(11) **EP 3 689 418 A1**

(12) **EUROPEAN PATENT APPLICATION**
published in accordance with Art. 153(4) EPC

(43) Date of publication:
**05.08.2020 Bulletin 2020/32**

(21) Application number: **18863567.6**

(22) Date of filing: **18.09.2018**

(51) Int Cl.:
**A61N 7/00** *(2006.01)*

(86) International application number:
**PCT/JP2018/034447**

(87) International publication number:
**WO 2019/065362 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2017 JP 2017189819**

(71) Applicants:
• **Nippon Medical School Foundation**
**Tokyo 113-8602 (JP)**
• **Pixie Dust Technologies, Inc.**
**Tokyo 1010061 (JP)**

(72) Inventors:
• **OGAWA Rei**
**Tokyo 113-8602 (JP)**
• **SUZUKI Hidenori**
**Tokyo 113-8602 (JP)**
• **SAKAI Atsushi**
**Tokyo 113-8602 (JP)**
• **WAKABAYASHI Nao**
**Tokyo 113-8602 (JP)**
• **TAKADA Hiroya**
**Tokyo 113-8602 (JP)**
• **HOSHI Takayuki**
**Tokyo 101-0041 (JP)**

(74) Representative: **Müller-Boré & Partner**
**Patentanwälte PartG mbB**
**Friedenheimer Brücke 21**
**80639 München (DE)**

(54) **ULTRASONIC TREATMENT APPARATUS**

(57) An ultrasonic therapy apparatus includes a plurality of ultrasonic transducers, a processor that identifies a position of an affected part and determines a focal point of ultrasonic waves radiated by the plurality of ultrasonic transducers based on the determined position of the affected part, and a drive circuit that generates a drive signal for driving the plurality of ultrasonic transducers at individual timings so that the ultrasonic waves are focused at the determined focal point.

[FIG. 7]

**Description**

[Technical Field]

The present invention relates to an ultrasonic therapy apparatus.

[Background Art]

It is known that the application of physical stimulation from the body surface of an animal has a therapeutic effect on a wound. This is because mechanical stress (for example, shear stress or pressure) generated inside vascular endothelial cells around the wound through the extracellular matrix by physical stimulation promotes angiogenesis or wound closure.
In particular, since ultrasonic activates fibroblasts, vascular endothelial cells, or leukocytes, it is known that the therapeutic effect on wounds is high.
For example, Japanese Patent Application Publication No. 2007-521053 discloses a technique of applying a physical stimulus to an affected part by irradiating an ultrasonic wave to the affected part to which a medicine is applied.

[Summary of Invention]

[Technical Problem]

In the treatment using physical stimulation by ultrasonic, the risk of infection during treatment becomes a problem. Infection during treatment not only increases the time to healing, but can also exacerbate the wound.
In Japanese Patent Application Publication No. 2007-521053, since a medicine is applied or sprayed to an affected part, there is a risk of infection when applying the medicine.
It is an object of the present invention to reduce the risk of infection in treatment with ultrasonic.

[Solution to Problem]

One aspect of the present invention is an ultrasonic therapy apparatus that includes
a plurality of ultrasonic transducers;
a processor,
identifying a position of an affected part;
determining a focal point of ultrasonic waves radiated by the plurality of ultrasonic transducers based on the identified position of the affected part; and
a drive circuit that generates a drive signal for driving the plurality of ultrasonic transducers at individual timings so that the ultrasonic waves are focused at the determined focal point.

[Advantageous Effects of Invention]

According to the present invention, the infection risk in the treatment using an ultrasonic wave can be reduced.

[Brief Description of Drawings]

FIG. 1 is a schematic diagram illustrating a configuration of an ultrasonic therapy apparatus according to an embodiment.
FIG. 2 is a block diagram showing functions of a controller shown in FIG. 1;
FIG. 3 is a schematic diagram illustrating a configuration of an ultrasonic radiation unit in FIG. 1;
FIG. 4 is an explanatory diagram of a method of determining a drive timing of the ultrasonic transducer in FIG. 3;
FIG. 5 is a schematic diagram of an operation example 1 of the phased array of FIG. 3;
FIG. 6 is a schematic diagram of operation example 2 of the phased array of FIG. 3;
FIG. 7 is a schematic diagram showing a contour of the present embodiment.
FIG. 8 is a flowchart of a process performed by the ultrasonic therapy apparatus according to the embodiment.
FIG. 9 is an explanatory diagram of a preparation process for starting treatment using the ultrasonic therapy apparatus of the present embodiment.
FIG. 10 is an explanatory diagram of step S101 in FIG. 8;

FIG. 11 is an explanatory diagram of the example 1.
FIG. 12 is an explanatory diagram of the example 1.
FIG. 13 is a diagram showing experimental results of example 1.
FIG. 14 is an explanatory diagram of the example 2.
FIG. 15 is a diagram showing experimental results of example 2.
FIG. 16 is an explanatory diagram of the example 3.
FIG. 17 is an explanatory diagram of the example 3.
FIG. 18 is an explanatory diagram of the example 5.
FIG. 19 is an explanatory diagram of the example 6.
FIG. 20 is a diagram showing experimental results of example 6.
FIG. 21 is an explanatory diagram of a comparative example of example 6.
FIG. 22 is a diagram showing experimental results of a comparative example of example 6.
FIG. 23 is an explanatory diagram of the example 8.
FIG. 24 is a view showing an experimental result of example 8.
FIG. 25 is a diagram showing experimental results of a comparative example of example 8.
FIG. 26 is a schematic view showing a contour of a variation 1.
FIG. 27 is a diagram illustrating a data structure of a parameter determination table according to the variation 1.

[Description of Embodiments]

Hereinafter, an embodiment of the present invention is described in detail based on the drawings. Note that, in the drawings for describing the embodiments, the same components are denoted by the same reference sign in principle, and the repetitive description thereof is omitted.

(1) Configuration of ultrasonic therapy apparatus

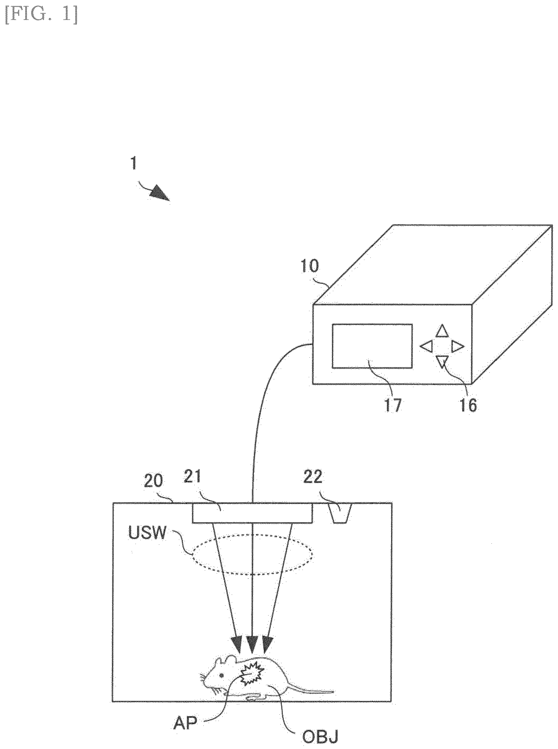

The configuration of the ultrasonic therapy apparatus according to the present embodiment is described. FIG. 1 is a schematic diagram illustrating a configuration of the ultrasonic therapy apparatus according to the present embodiment.

The ultrasonic therapy apparatus 1 in FIG. 1 is configured to treat an affected part AP of the treatment target OBJ by radiating the ultrasonic wave USW toward the treatment target OBJ. The OBJ to be treated is a human, an animal other than a human (eg, a mammal, a fish, a bird, an amphibian, or a reptile), or a plant.

The ultrasonic therapy apparatus 1 includes a controller 10 and an ultrasonic radiation unit 20.

The controller 10 is connected to the ultrasonic radiation unit 20. An operation unit 16 and a display unit 17 are arranged on one surface of the controller 10.

(1-1) Configuration of controller

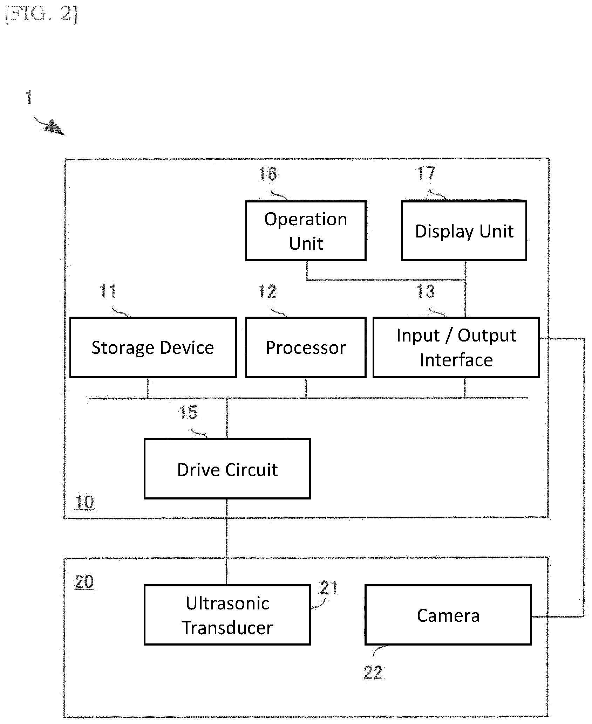

The configuration of the controller according to the present embodiment is described. FIG. 2 is a block diagram illustrating functions of the controller in FIG. 1.

As illustrated in FIG. 2, the controller 10 includes a storage device 11, a processor 12, an input / output interface 13, a drive circuit 15, an operation unit 16, and a display unit 17.

The memory 11 is configured to store a program and data. The memory 11 is, for example, a combination of a ROM (read only memory), a RAM (random access memory), and a storage (for example, a flash memory or a hard disk).

The programs include, for example, the following programs.

- OS (Operating System) program
- A driver application program for controlling the ultrasonic radiating unit 20

The data includes, for example, the following data.

- Database referred to in information processing
- Data obtained by executing an information processing (that is, an execution result of an information processing)

The processor 12 is configured to realize a function of the controller 10 by activating a program stored in the storage device 11. The processor 12 is an example of a computer.

The input / output interface 13 acquires an instruction of a user of the ultrasonic therapy apparatus 1 (for example, a doctor or a patient using the ultrasonic therapy apparatus 1) from the operation unit 16 and outputs information to the display unit 17. The input device is, for example, a keyboard, a pointing device, a touch panel, or a combination thereof.

The drive circuit 15 is configured to generate a drive signal for driving the ultrasonic wave radiating unit 20 under the control of the processor 12.

The operation unit 16 is configured to receive a user's instruction to the controller 10.

The display unit 17 is configured to display an image generated by the controller 10. The display unit 17 is a liquid crystal display.

(1-2) Configuration of ultrasonic radiation section

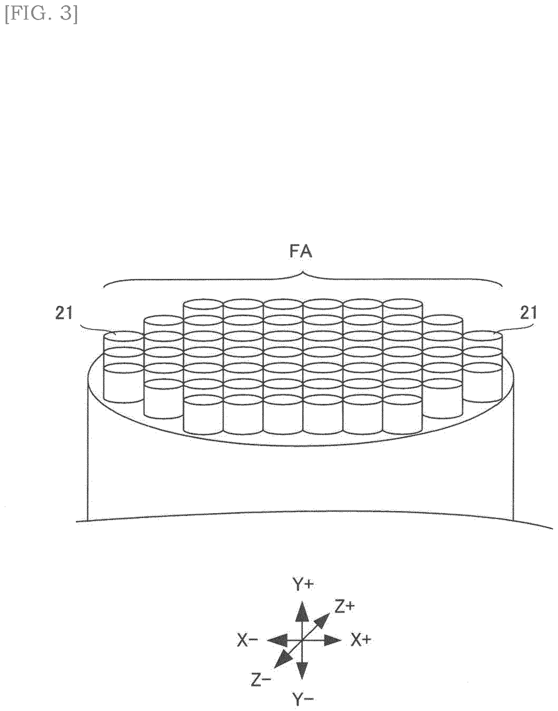

The configuration of the ultrasonic wave radiating unit of the present embodiment is described. FIG. 3 is a schematic diagram illustrating the configuration of the ultrasonic wave radiating unit in FIG. 1.

As shown in FIG. 2, the ultrasonic radiating unit 20 includes a plurality of ultrasonic transducers 21 and a camera 22.

The camera 22 is configured to capture an image and generate image data of the captured image.

As shown in FIG. 3, the plurality of ultrasonic transducers 21 form a phased array FA. The plurality of ultrasonic transducers 21 are arranged on an XZ plane (hereinafter, referred to as "array plane").

Each ultrasonic transducer 21 individually vibrates according to the drive signal generated by the drive circuit 15. Thereby, an ultrasonic wave is generated from each ultrasonic transducer 21. Ultrasonic waves radiated from the plurality of ultrasonic transducers 21 propagate in space and are focused at a focal point in space.

The controller 10 gives a phase difference to the ultrasonic waves radiated from each ultrasonic transducer 21c by individually controlling the drive timing of the plural ultrasonic transducers 21c. The position and number of focal points depend on this phase difference. That is, the controller 10 can change the position and number of focal points by controlling the phase difference.

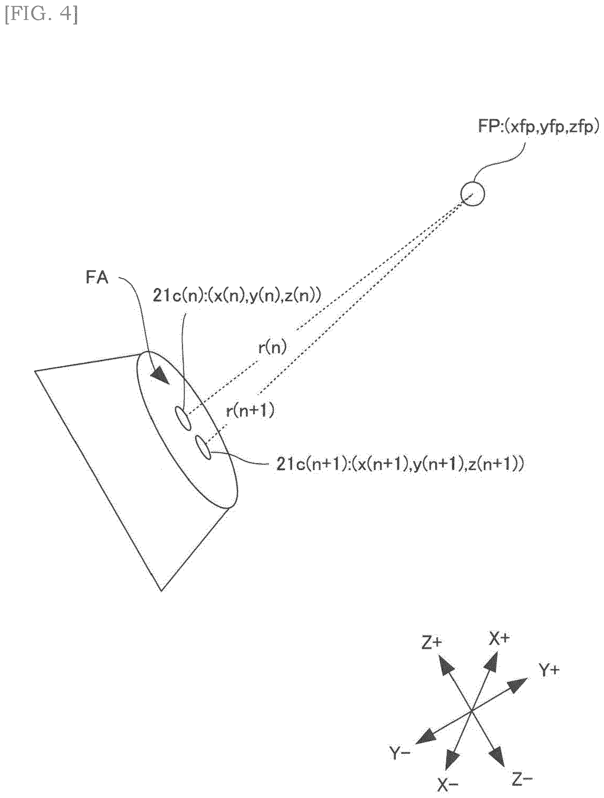

A method for forming a phase difference of ultrasonic waves according to the present embodiment is described. FIG. 4 is an explanatory diagram of a method for determining the drive timing of the ultrasonic transducers in FIG. 3.

The storage device 11 stores the coordinates (x (n), y (n), z (n)) of the ultrasonic transducer 21c (n) indicating the relative position of the ultrasonic transducer 21c (n) on the phased array FA with respect to the reference point (for example, the center) of the phased array FA. The symbol n is an identifier (positive integer) indicating the ultrasonic transducer 21c.

The processor 12 determines the focal coordinates (xfp, yfp, zfp) indicating the relative position of the focal point FP with respect to the reference point, as shown in FIG. 4.

The processor 12 calculated the distance r (n) between the ultrasonic transducer 21c (n) and the focal point FP based on the coordinates (x (n), y (n), z (n)) of the ultrasonic transducer 21c (n) stored in the storage device 11, and the focal coordinates (xfp, yfp, zfp).

The processor 12 calculates a time difference (hereinafter referred to as a "driving time difference") $\Delta T$ (n + 1) between the driving timing of the (n + 1) th ultrasonic transducer 21c (n + 1) and the driving timing of the nth ultrasonic transducer 21c (n) using Equation 1.

$$\Delta T \ (n + 1) = - \ r \ (n + 1) \ / \ c \qquad (\text{Equation 1})$$

· c: speed of sound

As described above, the processor 12 uses the focal coordinates (xfp, yfp, zfp) and the coordinates (x (n + 1), y (n + 1), z (n + 1)) stored in the storage device 11 to calculate the drive time difference $\Delta T$ (n + 1) of the ultrasonic transducer 21c (n + 1). The processor 12 supplies a drive signal to each ultrasonic transducer 21c (n + 1) according to the drive time difference $\Delta T$ (n + 1).

Each ultrasonic transducer 21c is driven according to the drive signal. The ultrasonic waves radiated from each ultrasonic transducer 21c have a phase difference corresponding to the drive time difference $\Delta T$ (n + 1), so that they are focused at the focal point FP.

(1-2-1) Operation example 1 of phased array (single focus)

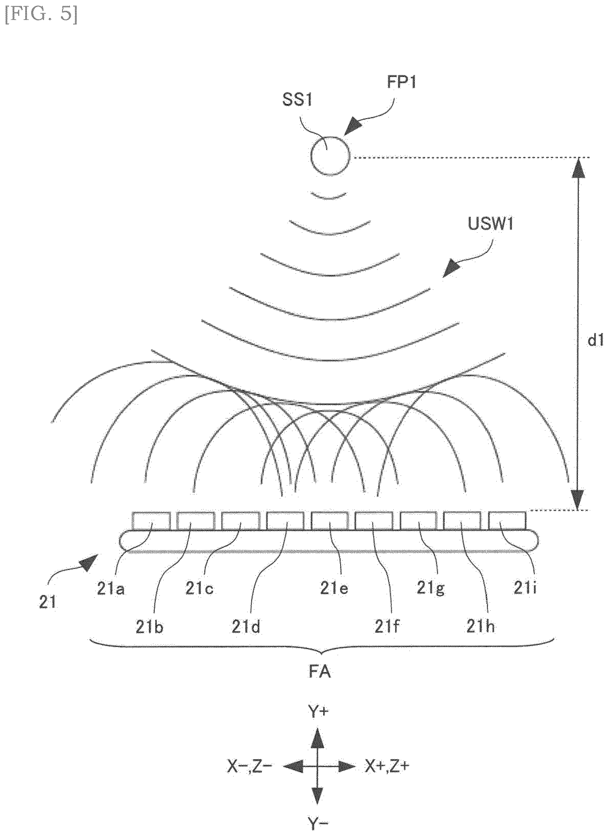

An operation example 1 of the phased array of the present embodiment is described. FIG. 5 is a schematic diagram of Operation Example 1 of the phased array of FIG. 3.

As shown in FIG. 5, in the operation example 1, the vibrations of the ultrasonic transducers 21a to 21i are temporally delayed in order from both ends to the center.

From the phased array FA, an ultrasonic wave USW1 having a phase difference corresponding to the time delay of the vibration is radiated. The ultrasonic wave USW1 is focused at a focal point FP1 separated from the phased array FA by a focal distance d1.

(1-2-2) Operation example 2 of phased array (double focus)

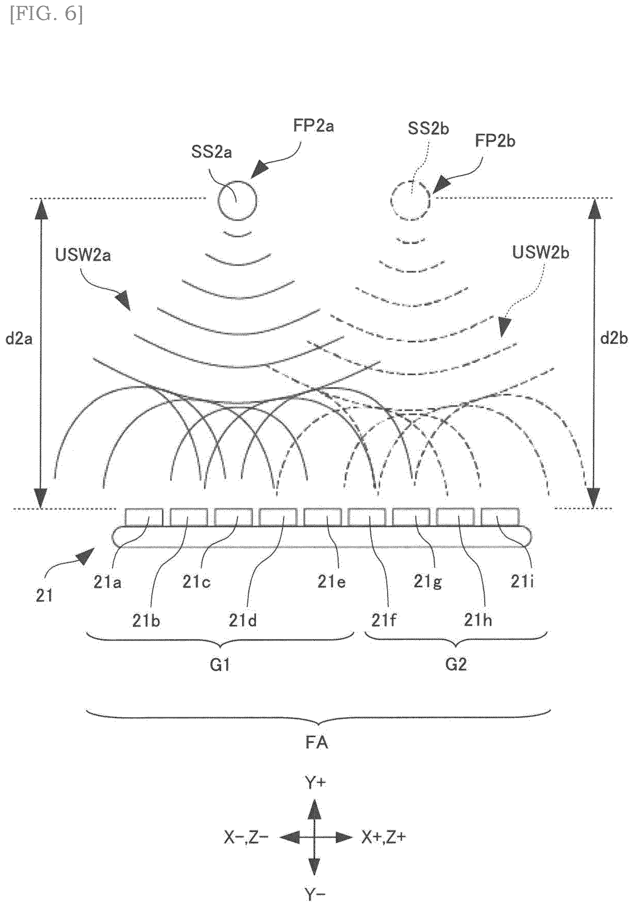

An operation example 2 of the phased array of the present embodiment is described. FIG. 6 is a schematic diagram of Operation Example 2 of the phased array of FIG. 3.

As shown in FIG. 6, in the operation example 2, the ultrasonic transducers 21a to 21i are divided into two groups G1 and G2. Group G1 includes ultrasonic transducers 21a to 21e. Group G2 includes ultrasonic transducers 21f to 21i.

The vibration of the group G1 (the ultrasonic transducers 21a to 21e) is temporally delayed in order from both ends to the center.

From the phased array FA, ultrasonic waves USW2a having a phase difference corresponding to the time delay of the vibration are radiated. The ultrasonic wave USW2a is focused at a focal point FP2a separated from the phased array FA by a focal distance d2a.

The group G2 (ultrasonic transducers 21f to 21i) is temporally delayed in order from both ends to the center.

From the phased array FA, ultrasonic waves USW2b having a phase difference corresponding to the time delay of the vibration are radiated. The ultrasonic wave USW2b is focused at a focal point FP2b separated from the phased array FA by a focal distance d2b.

The phased array FA can form three or more focal points.

(2) Overview of the present embodiment

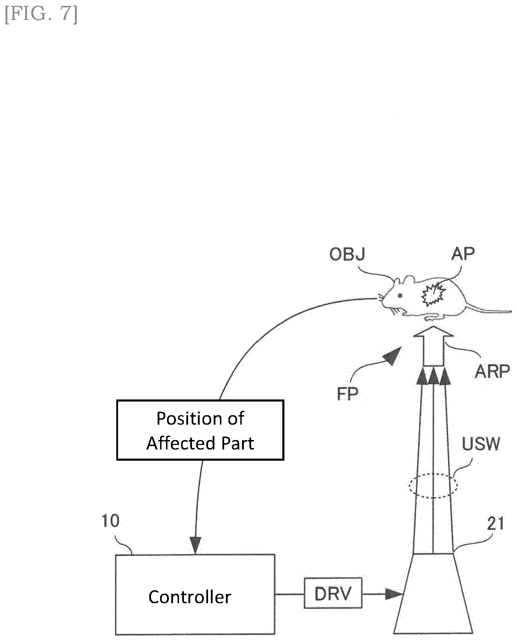

A contour of the present embodiment is described. FIG. 7 is a schematic diagram showing a contour of the present embodiment.

As shown in FIG. 7, when the position of the affected part AP is identified, the controller 10 determines the focus FP based on the position of the affected part AP. The controller 10 generates a drive signal DRV for driving the ultrasonic radiating unit 20 so as to radiate ultrasonic waves focused at the focal point FP.

The plurality of ultrasonic transducers 21 radiate a plurality of ultrasonic waves USW having a phase difference by individually driving according to the drive signal DRV generated by the controller 10.

The plurality of ultrasonic waves USW are focused at the focal point FP. The focused ultrasonic waves USW generates an acoustic radiation pressure ARP at the focal point FP. Since the focal point FP is determined based on the position of the affected part AP, the acoustic radiation pressure ARP is directly transmitted to the affected part AP. When the acoustic radiation pressure ARP is transmitted to the affected part AP, cell deformation occurs. This cell deformation increases the expression of genes involved in angiogenesis. This increase in gene expression accelerates angiogenesis in the affected part AP. As a result, the recovery of the affected part AP is promoted.

As described above, in the present embodiment, the controller 10 focuses the ultrasonic waves USW on the focal point FP determined based on the position of the affected part AP, so that the acoustic radiation pressure ARP generated at the focal point FP directly propagates to the affected part AP. The acoustic radiation pressure ARP is transmitted directly to the affected part AP without passing through a medium (for example, a spray medicine, a medicine, or a coupling gel). Therefore, there is no need to bring the ultrasonic radiation unit 20 into contact with the affected part AP, and it is not necessary to apply a medicine to the affected part AP. As a result, the risk of infection in treatment using ultrasonic can be reduced.

(3) Processing flow of the ultrasonic therapy apparatus

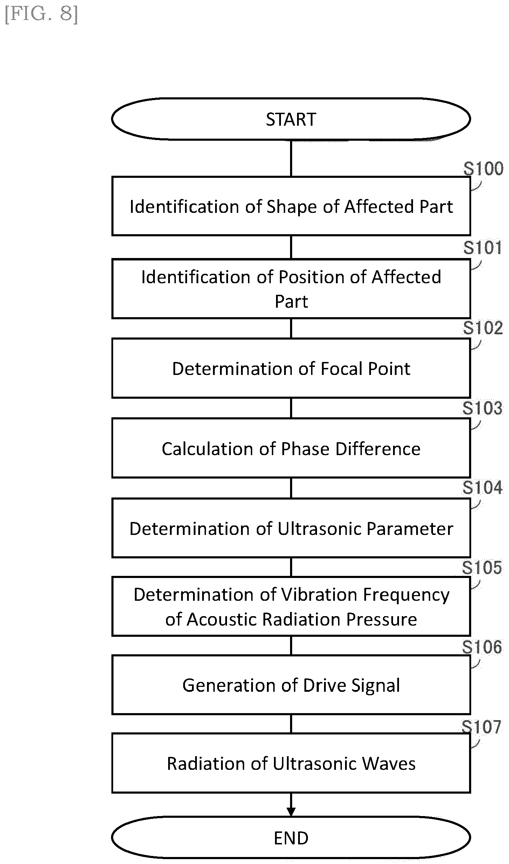

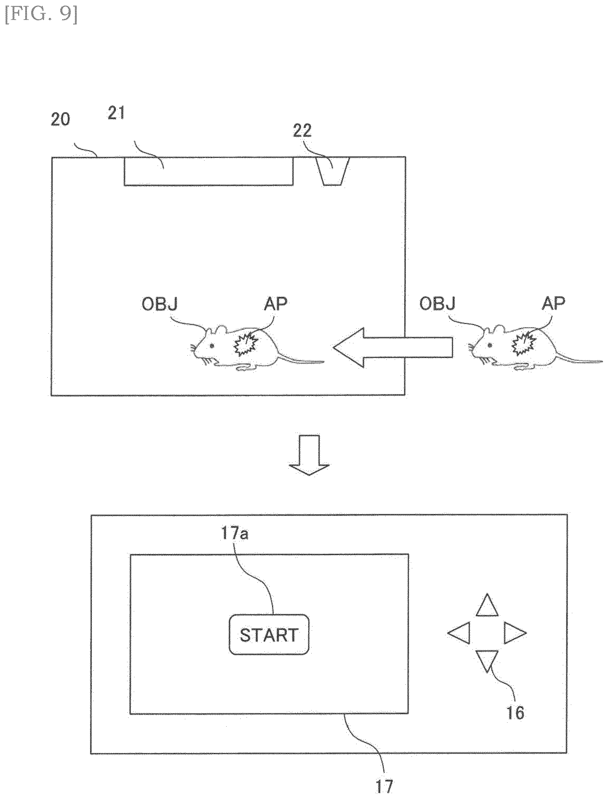

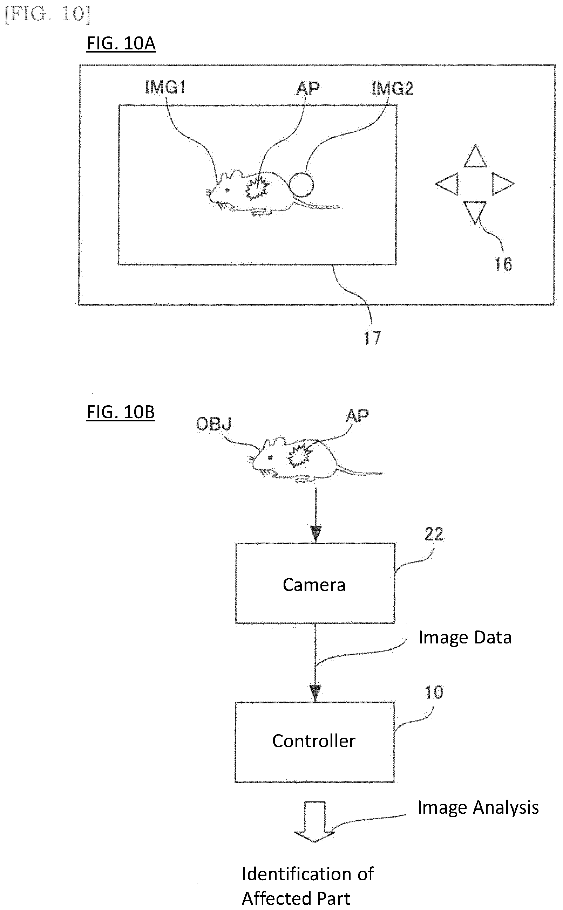

A processing flow of the ultrasonic therapy apparatus according to the present embodiment is described. FIG. 8 is a flowchart of processing of the ultrasonic therapy apparatus according to the present embodiment. FIG. 9 is an explanatory diagram of a preparation process for starting treatment using the ultrasonic therapy apparatus of the present embodiment. FIG. 10 is an explanatory diagram of step S101 in FIG. 8.

As shown in FIG. 9, a user (for example, a doctor) arranges a treatment target OBJ at a position facing the ultrasonic transducer 21 in the radiation direction of the ultrasonic transducer 21, and performs a predetermined operation (for example, a touch operation on the button object 17a displayed on the display unit 17) on the ultrasonic therapy apparatus 1. Then, the ultrasonic therapy apparatus 1 starts the processing in FIG. 8.

As shown in FIG. 8, the controller 10 executes identification of the shape of the affected part AP (S100).

Specifically, the camera 22 captures an image of the affected part AP, and generates image data of the captured image.

The processor 12 acquires the image data generated by the camera 22 via the input / output interface 13.

The processor 12 identifies the shape of the affected part AP by performing image analysis (e.g., feature amount analysis) on the acquired image data.

After step S100, the controller 10 executes the identification of the position of the affected part AP (S101).

A first example of step S101 will be described.

As shown in FIG. 10A, images IMG1 and IMG2 are displayed on the display unit 17. The image IMG1 is an image of treatment target OBJ captured by camera 22 in step S100. IMG2 is an image of a target marker.

The user operates the operation unit 16 while viewing the images IMG1 to IMG2 displayed on the display unit 17 in order to match the position of the target marker with the position of the affected part AP.

The processor 12 acquires, via the input / output interface 13, coordinate information corresponding to the position of the image IMG2.

The processor 12 identifies the relative position of the affected part AP with respect to the phased array FA in the three-dimensional space based on the acquired coordinate information.

A second example of step S101 will be described.

The processor 12 identifies the relative position of the affected part AP with respect to the phased array FA in the three-dimensional space by performing image analysis (for example, feature amount analysis) on the image data acquired in step S100.

After step S101, the controller 10 executes determination of the focus (S102).

Specifically, the processor 12 determines the number and arrangement of the focal point FP based on the shape of the affected part AP identified in step S100. As an example, when the affected part AP has a point shape, one focus FP is determined. As another example, when the affected part AP has a planar shape, a plurality of focal points FP are determined at positions included in a region surrounded by the contour of the affected part AP.

The processor 12 determines a distance from the center of the phased array FA to the focal point FP based on the relative position identified in step S101.

The processor 12 determines the angle of the focal point FP with respect to the normal of the phased array FA based on the relative position identified in step S101.

The three-dimensional coordinates of the focal point FP having the origin at the center of the phased array FA are determined by the distance and the angle.

After step S102, the controller 10 executes calculation of the phase difference (S103).

Specifically, the processor 12 calculate the phase difference based on the number, arrangement, distance, and angle of the focal point FP determined in step S102 such that the ultrasonic waves radiated by the plurality of ultrasonic transducers 21 focus at the focal points FP.

After step S103, the controller 10 executes determination of an ultrasonic parameter (S104).

Specifically, when the user operates the operation unit 16 to give a user instruction regarding the ultrasonic parameter to the controller 10, the processor 12 determines the ultrasonic parameter based on the user instruction.

The ultrasonic parameters include the following parameters.

- Ultrasonic radiation time
- Ultrasonic amplitude
- Ultrasonic modulation type (AM (Amplitude Modulation) or FM (Frequency Modulation))

After step S104, the controller 10 determines the vibration frequency of the acoustic radiation pressure (S105).

Specifically, when the user operates the operation unit 16 to give a user instruction regarding the vibration frequency of the acoustic radiation pressure to the controller 10, the processor 12 determines the vibration frequency based on the user instruction. The vibration frequency is, for example, a value between 0 and 100 Hz.

After step S105, the controller 10 executes generation of a drive signal (S106).

Specifically, the processor 12 individually determines the drive timing of each of the plurality of ultrasonic transducers 21 based on the phase difference calculated in step S103.

The processor 12 generates a drive signal based on the ultrasonic parameters determined in Step S104 and the vibration frequency determined in Step S105. The drive signal has, for example, a pulse waveform or a sine waveform. If the signal waveform of the drive signal is a rectangular wave, the pulse width is determined based on the radiation time determined in step S104. The pulse amplitude is determined based on the amplitude determined in step S104. The pulse frequency is determined based on the vibration frequency determined in step S105. If the signal waveform of the drive signal is a sine waveform, the wavelength is determined based on the radiation time determined in step S104. The amplitude is determined based on the amplitude determined in step S104. The frequency is determined based on the vibration frequency determined in step S105.

The drive circuit 15 outputs a drive signal to each ultrasonic transducer 21 in accordance with the drive timing of each ultrasonic transducer 21.

After step S106, the ultrasonic wave radiating unit 20 executes ultrasonic wave radiation (S107).

Specifically, each ultrasonic transducer 21 radiates an ultrasonic wave USW according to the drive signal output in step S106. The timing at which the drive signal is output to each ultrasonic transducer 21 is determined by the drive timing based on the phase difference calculated in S103. Therefore, the ultrasonic waves USW radiated from the plural ultrasonic transducers 21 have the phase difference calculated in S103.

The ultrasonic wave USW radiated from each ultrasonic transducer 21 is focused at the focal point FP determined based on the position of the affected part AP. The ultrasonic wave USW focused at the focal point FP generates an acoustic radiation pressure ARP (FIG. 7) at the focal point FP. This acoustic radiation pressure ARP is transmitted directly to the affected part AP. When the acoustic radiation pressure ARP is transmitted to the affected part AP, the expression of genes related to angiogenesis increases. This increase in gene expression accelerates angiogenesis in the affected part AP. As a result, the recovery of the affected part AP is promoted.

(4) Example

An example of the present embodiment is described.

(4-1) Example 1 (Promotion of wound closure by acoustic radiation pressure of ultrasonic waves)

Example 1 of the present embodiment is described. Example 1 is an example relating to promotion of wound closure by acoustic radiation pressure ARP of ultrasonic waves.

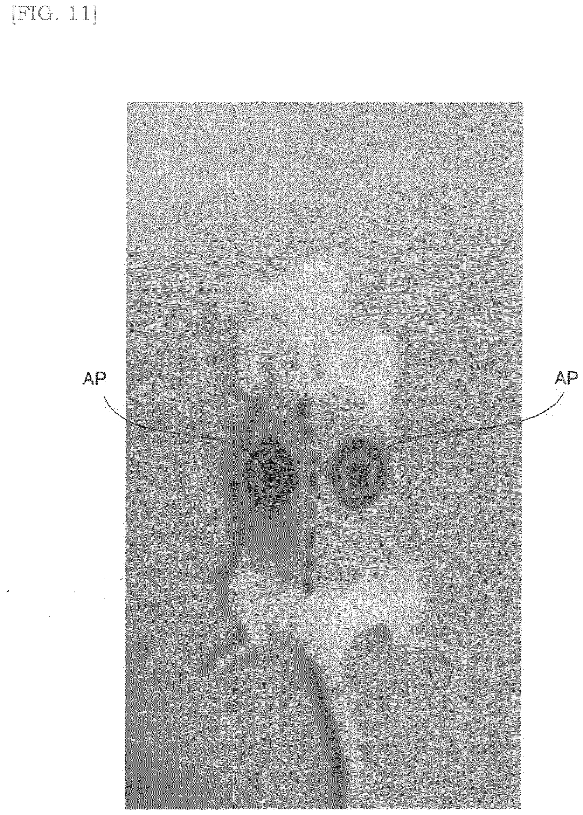

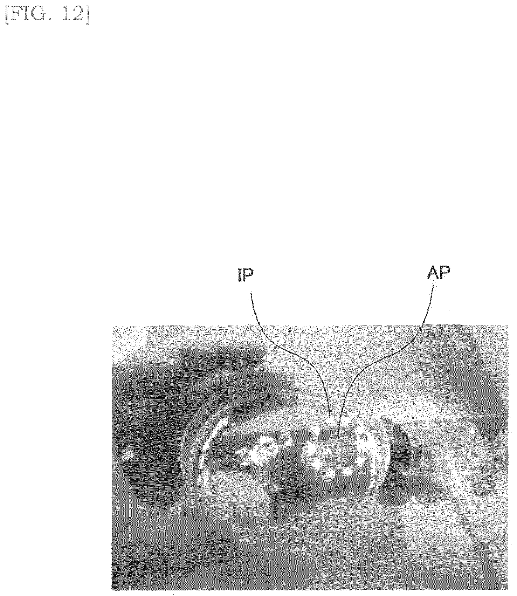

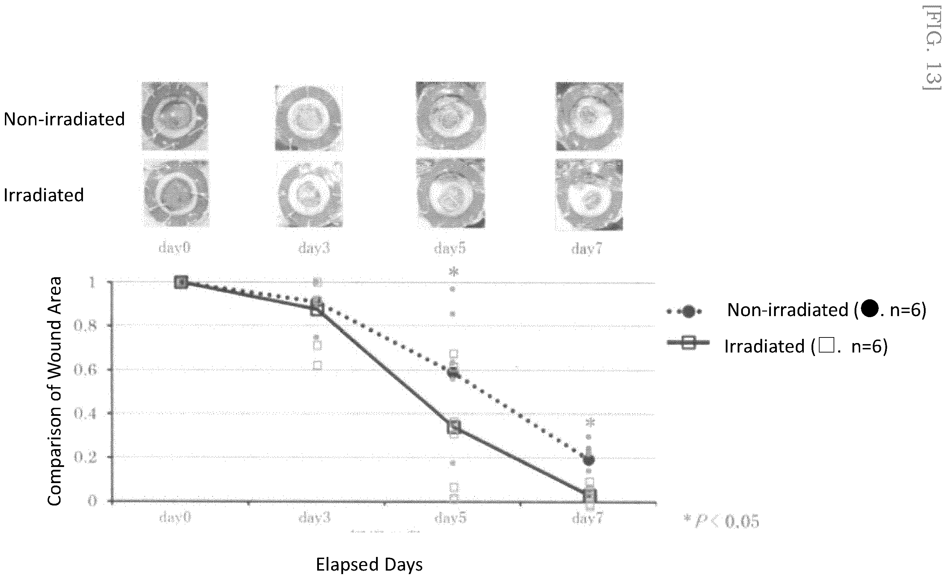

A normal wound model was prepared for normal mice by the following procedure. First, under an inhalation anesthesia using isoflurane, hair was removed with an electric razor for animals and a depilatory cream. Subsequently, under the microscope observation, two full-layer defect wound APs having a diameter of about 6. 5 mm were formed on the skin fascia symmetrically to the midline of the back (FIG. 11). A doughnut-shaped anti-shrink silicone ring was attached around the wound to prevent extension and deformation of the wound due to body movement, and the wound was coated with a water vapor-permeable transparent film (7 $\mu$m thick wound dressing film) to prevent the wound from drying. The irradiation position IP to the wound was confirmed (FIG. 12), and non-contact / periodic pressure stimulation (10 Hz, 90. 6 Pa, 1 hour / day, 3 consecutive days) was applied to one wound AP by acoustic radiation pressure ARP. The impact was evaluated. For wound closure evaluation, the area of the non-epithelial site was calculated from the wound edge using image analysis software (ImageJ), and statistical processing was applied. As a result, the closing rate after 7 days reached 97% in the irradiated side wound AP, whereas the closing rate after 7 days in the non-irradiated side wound AP was 79%. That is, it was confirmed that the wound closure on the irradiated side was significantly faster than that on the non-irradiated side (FIG. 13).

(4-2) Example 2 (Increase in collagen production by ultrasonic acoustic radiation pressure)

Example 2 of the present embodiment is described. Example 2 is an example of the increase in collagen production by the acoustic radiation pressure ARP of ultrasonic.

It is known that granulation formation by collagen augmentation is important in the process of wound healing. Therefore, observing the state of collagen fiber (collagen) production using a test mouse is an index of wound healing.

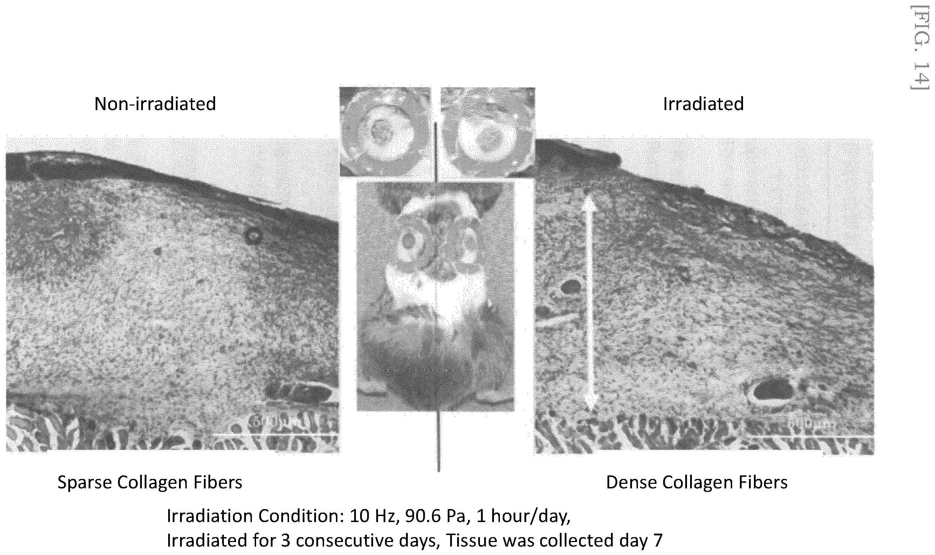

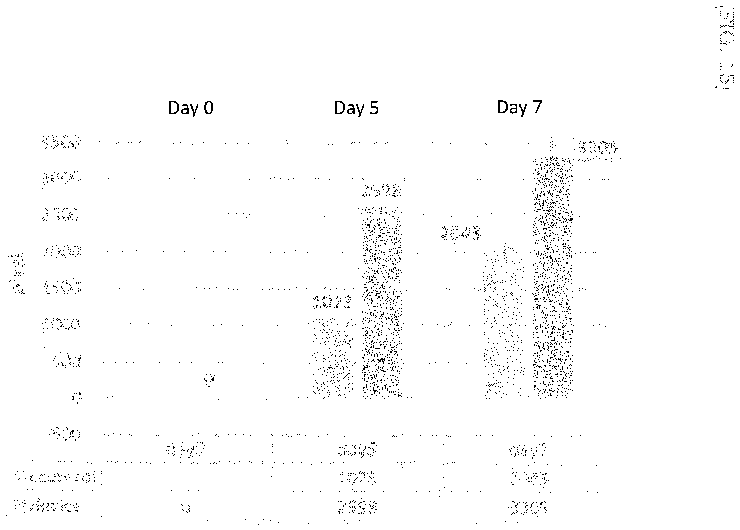

As in Example 1, non-contact / periodic pressure stimulation by acoustic radiation pressure ARP (10 Hz, 90.6 Pa, 1 hour / day, 3 consecutive days) was applied to the wound AP on one side of the test mouse where the wounds AP were created in two places. Wound tissue pieces were collected 5 days and 7 days after the start of applying. Frozen sections were prepared from the collected tissue pieces, and fixed with 4% paraformaldehyde. For quantification of collagen production, Masson trichrome staining was performed to specifically stain collagen, and it was confirmed that collagen fibers were stained green to pale blue (FIG. 14). From the stained images, collagen fibers were densely observed in all layers of the granulation on the apparatus irradiated side. Furthermore, the ratio of the luminance of each pixel of the collagen staining to the entire section image was analyzed with image editing software (Photoshop (registered trademark)). Comparing the average number of pixels of the stained image after 5 days, it was 2598 on the irradiated side, whereas it was 1073 on the non-irradiated side. Collagen production increased 2. 4-fold on the irradiated side compared to the non-irradiated side (FIG. 15). Further, when the average number of pixels of the stained image after 7 days was compared, it was 3305 on the irradiated side, while it was 2043 on the non-irradiated side. Collagen production increased 1. 6-fold on the irradiated side compared to the non-irradiated side (FIG. 15). This is considered to be due to the fact that fibroblasts migrated and gathered at the wound site due to the acoustic radiation pressure ARP of the ultrasonic wave to produce collagen fibers (collagen).

(4-3) Example 3 (Promotion of angiogenesis by acoustic radiation pressure of ultrasonic waves)

Example 3 of the present embodiment is described. Example 3 is an example of promoting angiogenesis by the acoustic radiation pressure ARP of ultrasonic waves.

In the process of wound healing, the formation of capillaries for supplying nutrients and oxygen to the wound occurs. Therefore, evaluating the state of angiogenesis is an index of wound healing. Therefore, new blood vessels were evaluated using test mice.

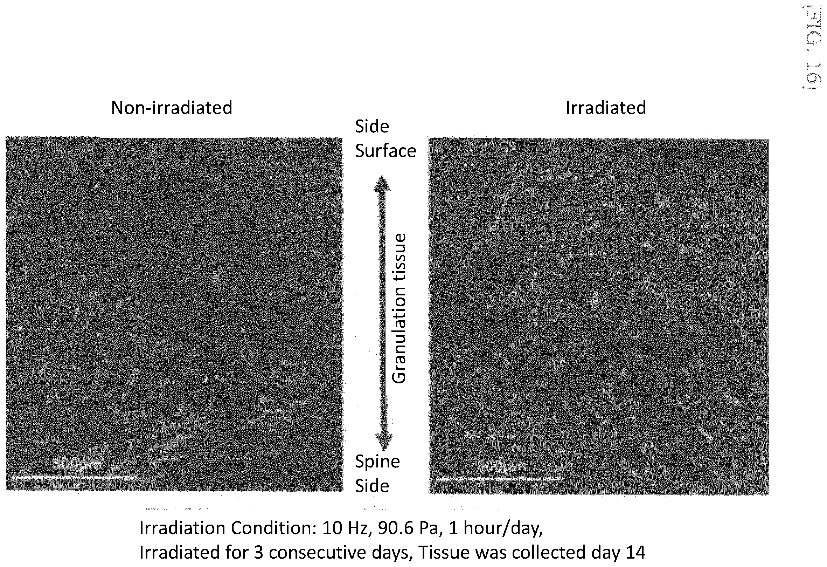

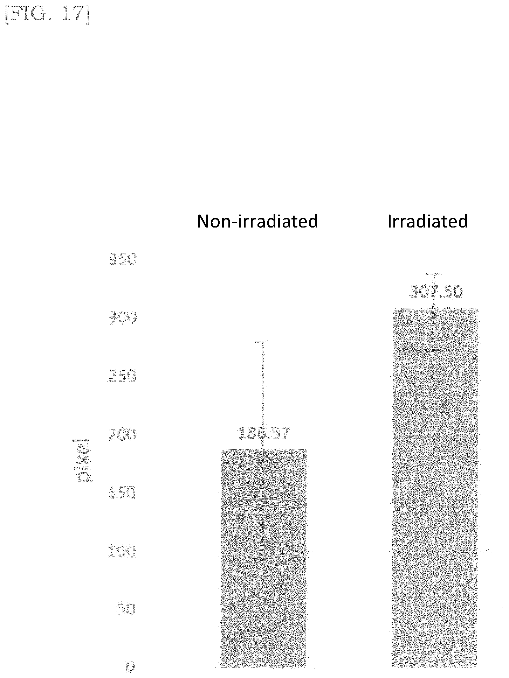

As in Example 1, non-contact / periodic pressure stimulation by acoustic radiation pressure ARP (10 Hz, 90.6 Pa, 1 hour / day, 3 consecutive days) was applied to the wound AP on one side of the test mouse where the wounds AP was created in two places. 14 days after the start of the applying, wound tissue pieces were collected respectively. The collected wound tissue section was embedded using an OCT compound, and then a frozen section was prepared. The section was fixed with 4% paraformaldehyde, and vascular endothelial cells were immunostained with an anti-CD31 antibody. From the immunostaining images, CD31-positive blood vessels were observed over the entire wound at the surface of the wound on the apparatus irradiated side (FIG. 16). The luminance of each pixel of CD31-positive cells obtained by immunostaining was analyzed with image editing software (Photoshop (registered trademark)). Comparing the average number of pixels of the immunostained image, it was 307.50 on the irradiated side, whereas it was 186.57 on the non-irradiated side. Angiogenesis increased 1. 6-fold on the irradiated side compared to the non-irradiated side (FIG. 17). This is considered to be because fibroblasts migrated and gathered in the wound by the acoustic radiation pressure ARP of the ultrasonic, collagen fibers (collagen) were produced, and subsequently angiogenesis was promoted.

(4-4) Example 4 (Optimal frequency of acoustic radiation pressure of ultrasonic waves)

Example 4 of the present embodiment is described. Example 4 is an example of the optimum frequency of the acoustic radiation pressure of the ultrasonic wave.

The following experiment was performed to determine the optimal frequency of the acoustic radiation pressure ARP for wound healing.

As in Example 1, non-contact, periodic pressure stimulation by acoustic radiation pressure ARP (90.6 Pa, 1 hour / day, 3 consecutive days) was to one side of the wound AP of a test mouse in which wounds were created in two places.. There were four different patterns of acoustic radiation pressures ARP (four types of 0 Hz, 1 Hz, 10 Hz, and 100 Hz). Three, five, and ten days after the start of applying, wound tissue sections were collected, frozen sections were prepared from the collected wound tissue sections, and fixed with 4% paraformaldehyde. Hematoxylin and eosin (HE) staining and Masson chrome staining were performed. The degree of contraction of the wound, the disappearance of inflammatory cells estimated from the degree of infiltration, and the thickness of the collagen tissue of the granulation are visually determined from the stained image of the irradiated side were compared with the non-irradiated side were compared (Table 1). As a result of determining the optimal frequency in the wound healing process, an appropriate tendency of 100 Hz < 0 Hz < 1 Hz <= 10 Hz was observed.

[Table 1]

| Macroscopic Findings (H-E Stain, etc.) | Frequency | | | |
|---|---|---|---|---|
| | 0 Hz | 1 Hz | 10 Hz | 100 Hz |
| Wound Contraction | Normal | Very Early | Early | Normal |
| Disappearance of Inflammatory Cells | Early | Early | Very Early | Late |
| Thickness of Collagen Tissue of Granulation | Thick | Thick | Very Thick | Thin |

(4-5) Example 5 (Micro-deformation of vascular endothelial cells by acoustic radiation pressure of ultrasonic waves)

Example 5 of the present embodiment is described. Example 5 is an example of microdeformation of vascular endothelial cells by acoustic radiation pressure ARP of ultrasonic waves.

The following experiment was conducted in order to investigate the effect of ultrasonic acoustic radiation pressure ARP on vascular endothelial cells physically.

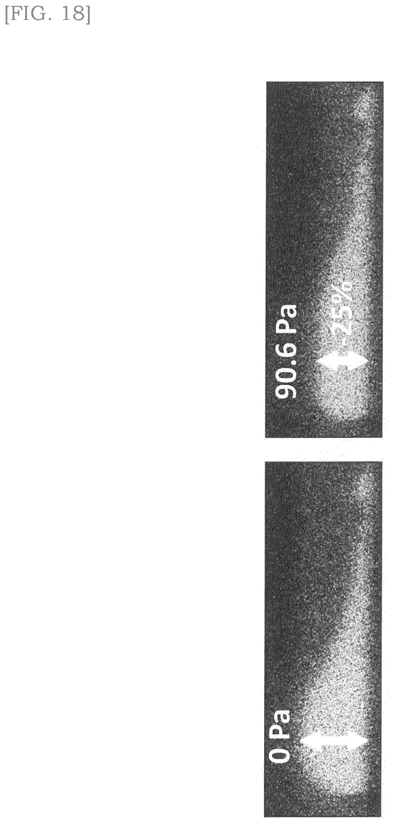

Calcein AM is a fluorescent dye capable of visually staining cell morphology that is difficult to see with the naked eye through the cell membrane of living cells. The Calcein AM is loaded (1 hour) to human microvascular endothelial cells (HMEC-1 cells) cultured on a type I collagen gel for 3 to 6 hours. Next, fluorescence tomographic observation was performed from the top surface (apical position) of the HMEC-1 cells by a real-time imaging method using a confocal laser scanning microscope (LSM 510/710) while applying pressure stimulus (90.6 Pa). Thereby, the amount of cell deformation due to the acoustic radiation pressure ARP of the ultrasonic wave could be visually analyzed. Cells were compressed from the top surface loaded with acoustic radiation pressure ARP and flattened $25 \pm 5\%$ (FIG. 18).

(4-6) Example 6 (High-frequency Ca$^{2+}$ oscillation of vascular endothelial cells by acoustic radiation pressure of ultrasonic waves)

Example 6 of the present embodiment is described. Example 6 is an example of high-frequency Ca$^{2+}$ oscillation of vascular endothelial cells by ultrasonic acoustic radiation pressure ARP.

The following experiment was conducted in order to examine the fluctuation of intracellular calcium ion (Ca$^{2+}$) concentration caused by the acoustic radiation pressure ARP of ultrasonic waves.

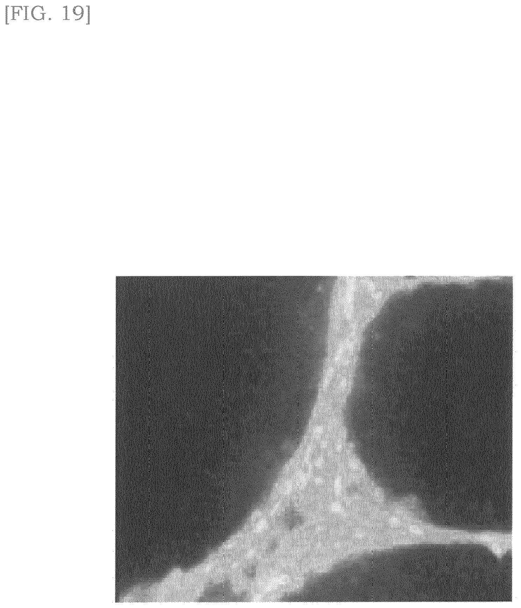

HMEC-1 cells were seeded on Matrigel to create a vascular-like network of HMEC-1 cells in a short time. After 3 to 6 hours, Fluo-8 AM was loaded (1 hour) as a fluorescent indicator of Ca$^{2+}$ dynamics, and periodic pressure stimulation (10 Hz, 90. 6 Pa) by acoustic radiation pressure ARP was applied to the top surface of the cells ((FIG. 19). This was observed by the real-time imaging method.

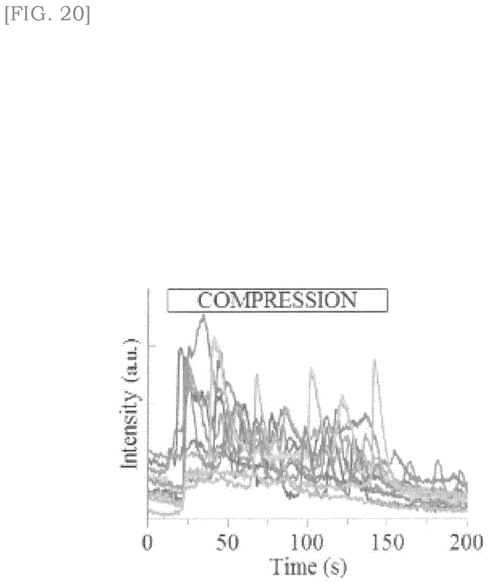

Ca$^{2+}$ oscillation occurred intracellularly at high frequency (up to 7 times / min) immediately after loading. Ca$^{2+}$ oscillation is an oscillation phenomenon in which the concentration of Ca$^{2+}$ repeatedly rises and falls in a short time. On the other hand, when the periodic pressure stimulation was stopped, intracellular Ca$^{2+}$ oscillation attenuated to the same level as before the application of the periodic pressure stimulation (FIG. 20).

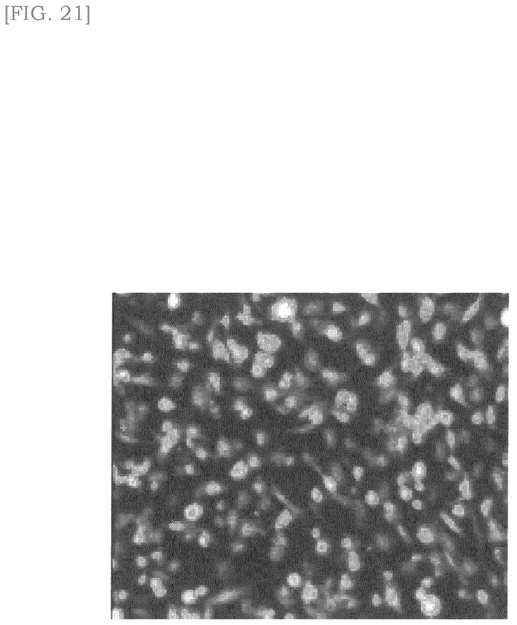

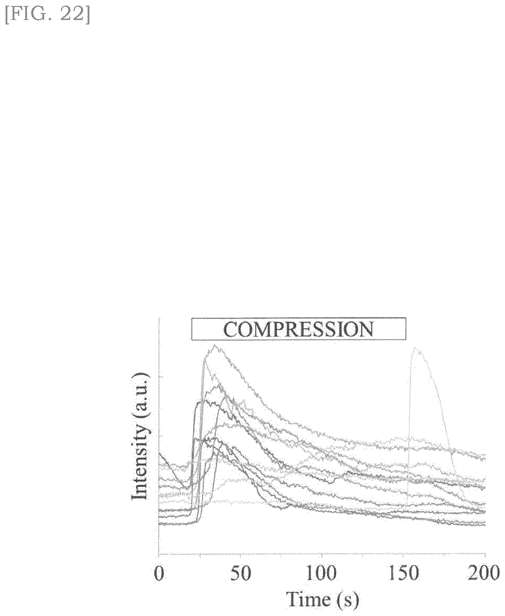

As a comparative example of the process of forming a blood vessel-like network, intracellular Ca$^{2+}$ dynamics were observed by real-time imaging under the conditions of the growth process in which HMEC-1 cells were cultured on a collagen gel (FIG. 21). As a result, although the Ca$^{2+}$ concentration changed due to the periodic pressure stimulation, it was confirmed that the Ca$^{2+}$ concentration did not repeat rising and falling in a short time as shown in FIG. 20 (FIG. 22).

(4-7) Example 7 (Change in gene expression related to angiogenesis due to acoustic radiation pressure of ultrasonic waves)

Example 7 of the present embodiment is described. Example 7 is an example of a change in gene expression related to angiogenesis due to the acoustic radiation pressure ARP of ultrasonic.

We analyzed how the acoustic radiation pressure of ultrasonic, which causes cell deformation and high-frequency Ca$^{2+}$ oscillation, changes gene expression in HMEC-1 cells.

As in Example 6, HMEC-1 cells were seeded on Matrigel, which can create a vascular network in a short time. A cyclic pressure stimulus (10 Hz / 90.6 Pa / 1 hour) is applied from the top surface of the cell (apical position) to induce a state in which high-frequency Ca$^{2+}$ oscillation occurs in the cell, and then the cell is cultured in an incubator for 24 hours. RNA was extracted from irradiated and unirradiated cells, respectively, using standard protocols. As a result of comprehensive analysis of gene expression using a microarray (Agilent Technologies), more than 29,000 gene expression information was obtained. From these, genes with high gene expression variation ratio (FC> 1.5) and high p-value (p <0.05) in the irradiation group compared to the non-irradiation group were narrowed down, and the expression of genes related to angiogenesis such as Hey1, Hey2, Nrarp, EphB4, and ephrinB2 was increased (Table 2). In particular, Hey1 and Hey2, downstream transcription regulators of the Notch signal, which are thought to play an important role in angiogenesis, increased by 4.6 and 3.5 fold, respectively. Nrarp that regulates vascular density in angiogenesis, and the membrane proteins ephrinB2 and EphB4 expressed by arterial endothelial cells, and venous endothelial cells also increased, respectively.

[Table 2]

| Gene | Hey1 | Hey2 | Nrarp | EphB4 | ephrinB2 |
|------|------|------|-------|-------|----------|
| FC | 4.64 | 3.47 | 1.74 | 1.65 | 1.57 |

(4-8) Example 8 (Promotion of formation of vascular-like network of vascular endothelial cells by pressure stimulation from top surface of cells)

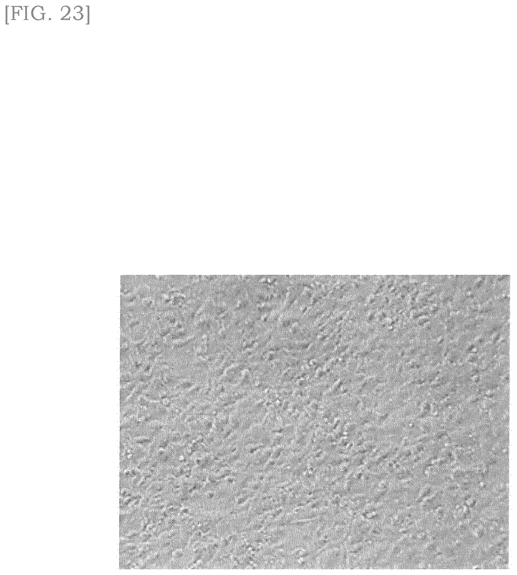

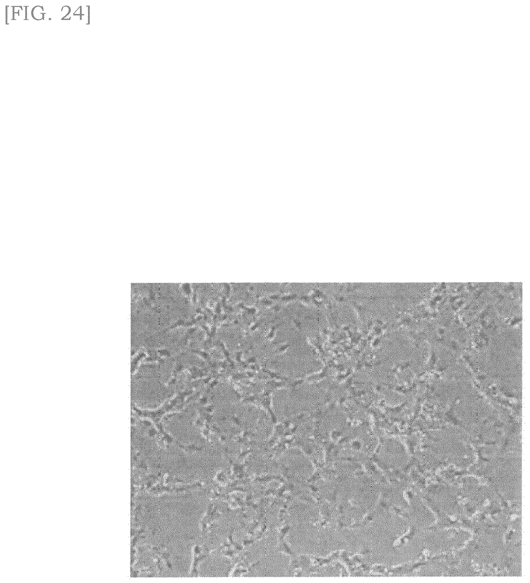

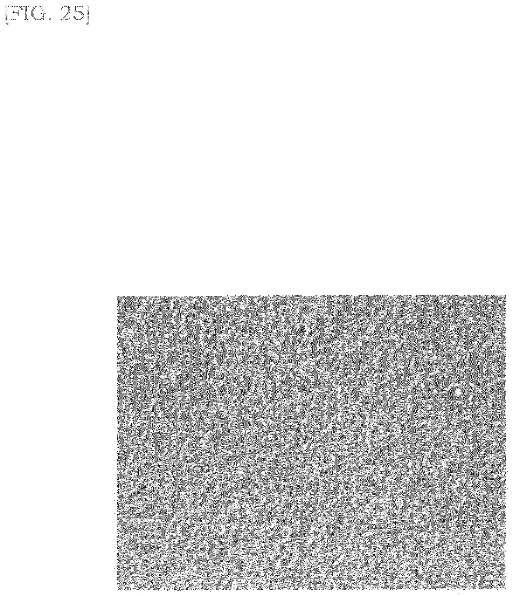

Example 8 of the present embodiment is described. Example 8 is an example of promoting formation of a vascular-like network of vascular endothelial cells by pressure stimulation from the cell top surface. FIG. 23 is an explanatory diagram of the example 8. FIG. 24 is a diagram illustrating experimental results of Example 8. FIG. 25 is a diagram illustrating experimental results of a comparative example of Example 8.

From the results obtained in Examples 1 to 7, we thought that moderate periodic pressure stimulation directly causes microdeformation of cells, which triggers the regulation of proliferation and differentiation of vascular endothelial cells and initiates the formation of a vascular-like network. We thought that, in the process of the formation of a vascular-like network, high frequency Ca$^{2+}$ oscillation occurred, activating endothelial function and promoting angiogenesis. Therefore, we focused on the behavior of HMEC-1 cells cultured on type I collagen gel closer to the biological environment.

The HMEC-1 cells cultured under these conditions proliferate like a cobblestone and show a planar structure (FIG. 23). When a continuous pressure stimulus (90. 6 Pa) was applied from the top surface of the cell (apical position), a vascular-like network structure was formed in 24 hours (FIG. 24). Further, when the extracellular solution which is considered to contribute to the change in intracellular $Ca^{2+}$ concentration was replaced with a low $Ca^{2+}$ concentration (0. 07 mM) solution and the cells were cultured. As shown in FIG.25, vascular-like structure was not formed, and cell proliferation was exhibited.

(4-9) Example 9 (Constant pressure stimulation by acoustic radiation pressure of ultrasonic waves)

Example 9 of the present embodiment is described. Example 9 is an example of constant pressure stimulation by the acoustic radiation pressure ARP of ultrasonic waves.

In the same manner as in Example 1, one side of each of the test mice in which the wound AP was created was applied with a constant non-contact pressure stimulus (0 Hz, 90.6 Pa, 1 hour / day, 3 consecutive days). Seven days after the start of applying (4 days after applying), visual observation of wound closure and epithelialization revealed that healing was faster than non-irradiated controls.

(5) Variation

variations will be described.

(5-1) Variation 1

Variation 1 will be described. The variation 1 is an example in which at least one of a vibration frequency and an ultrasonic parameter is determined according to a treatment target.

(5-1-1) contour of variation Example 1

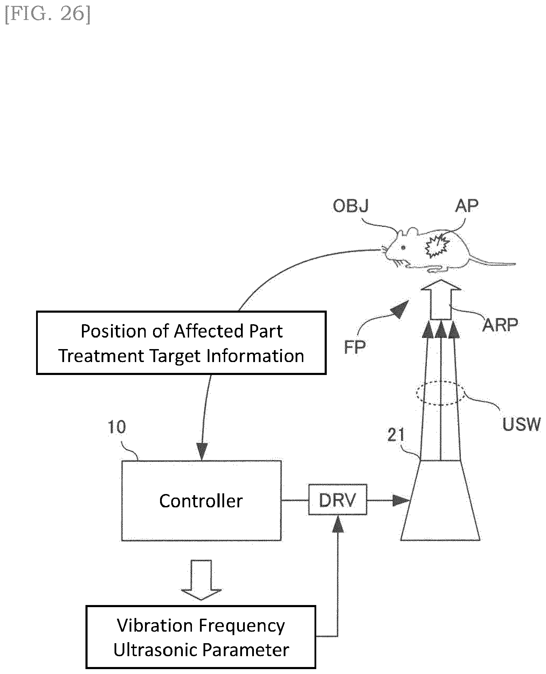

An contour of the variation 1 is described. FIG. 26 is a schematic diagram showing a contour of the variation 1.

As shown in FIG. 26, the controller 10 of the variation 1 differs from the present embodiment (FIG. 7) in that the controller 10 of the variation 1 determines the vibration frequency and the ultrasonic parameter based on the treatment target information regarding the treatment target, and generates the drive signal DRV based on the determined vibration frequency and the ultrasonic parameter.

The plurality of ultrasonic waves USW have waveforms according to the vibration frequency and the ultrasonic parameters. Since the vibration frequency and the pressure intensity of the acoustic radiation pressure ARP generated at the focal point FP depend on the vibration frequency and the ultrasonic parameters, the acoustic radiation pressure ARP corresponding to the treatment target information is directly transmitted to the affected part AP.

(5-1-2) Database

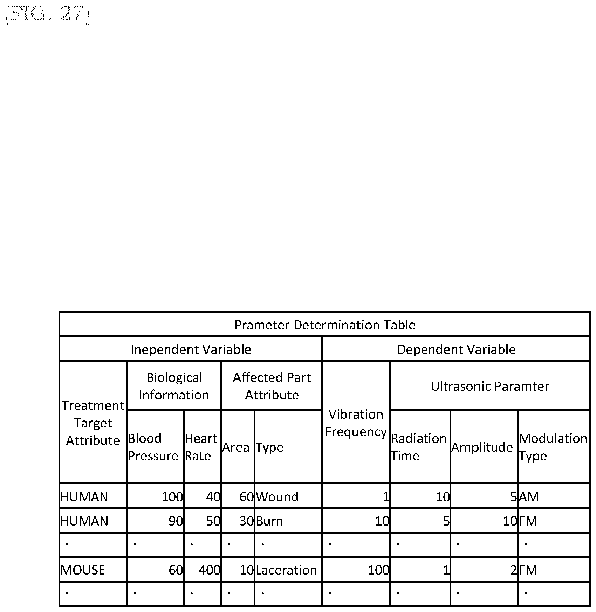

The database of the variation 1 is described. FIG. 27 is a diagram illustrating a data structure of a parameter determination table according to the variation 1.

As shown in FIG. 27, the parameter determination table stores information for determining a vibration frequency and an ultrasonic parameter according to a treatment target.

The parameter determination table includes an "independent variable" field and a "dependent variable" field.

In the "independent variable" field, an independent variable for determining a vibration frequency and an ultrasonic parameter is stored. The "independent variable" field includes a "treatment target attribute" field, a "biological information" field, and an "affected part attribute" field.

The "treatment target attribute" field stores treatment target attribute information related to a treatment target attribute (for example, a biological species).

The "biological information" field stores biological information regarding a living body to be treated. The "biological information" field includes a "blood pressure" field and a "heart rate" field.

The "blood pressure" field stores information on the blood pressure of the treatment target.

The "heart rate" field stores information on the heart rate of the treatment target.

The "affected part attribute" field stores affected part attribute information on the attribute of the affected part AP. The "affected part attribute" field includes an "area" field and a "type" field.

The "area" field stores information on the area of the affected part AP.

The "type" field stores information on the type of the affected part AP (for example, a wound, a burn, or a laceration).

The "dependent variable" field stores a dependent variable that depends on the variable in the "independent variable" field. The "dependent variable" field includes a "vibration frequency" field and an "ultrasonic parameter" field.

The "vibration frequency" field stores information on the vibration frequency.

In the "ultrasonic parameter" field, information on the ultrasonic parameter is stored. The "ultrasonic parameter" field includes a "radiation time" field, an "amplitude" field, and a "modulation type" field.

The "radiation time" field stores information on the radiation time of the ultrasonic wave.

The "amplitude" field stores information on the amplitude of the ultrasonic wave.

The "modulation type" field stores information on the modulation type of the ultrasonic wave.

(5-1-3) Processing flow of the ultrasonic therapy apparatus

The processing flow of the ultrasonic therapy apparatus according to the variation 1 is described.

A first example of the processing flow of the ultrasonic therapy apparatus according to the variation 1 is described.

When the user operates the operation unit 16 to provide all the independent variables (for example, the treatment target attribute, the blood pressure and the heart rate of the treatment target, and the area and type of the affected part AP) to the controller 10, in step S104 (FIG. 8), the processor 12 determines the dependent variable dependent on the independent variable given by the user as the ultrasonic parameter with reference to the parameter determination table (FIG. 27).

A second example of the processing flow of the ultrasonic therapy apparatus according to the variation 1 is described.

When the user operates the operation unit 16 to give some independent variables (for example, treatment target attributes) to the controller 10, the processor 12 acquires information on the blood pressure and heart rate of the treatment target (not shown) from the measurement apparatus (not shown) attached to the treatment target.

In step S100, the processor 12 performs image analysis (e.g., feature amount analysis) on the acquired image data to identify the area and type of the affected part AP in addition to the shape of the affected part AP.

In step S104 (FIG. 8), the processor 12 refers to the parameter determination table (FIG. 27) and determines the dependent variable dependent on these independent variables (the treatment target attribute given by the user, the blood pressure and heart rate acquired from the measurement apparatus, and the area and type of the affected part AP obtained by the image analysis) as the ultrasonic parameter.

According to the variation 1, the controller 10 focuses the ultrasonic waves USW corresponding to the vibration frequency and the ultrasonic parameter determined based on the treatment target information. Thereby, the optimal acoustic radiation pressure ARP for the treatment target can be transmitted to the affected part AP.

(5-2) Variation 2

Variation 2 will be described. The variation 2 is an example in which at least one of the vibration frequency and the ultrasonic parameter is dynamically changed.

The controller 10 of the variation 2 repeatedly executes the processing of steps S101 to S103 during the treatment. Therefore, when the position of the affected part AP moves, the phase difference calculated in step S103 changes. As a result, the drive signal output in step S106 changes according to the movement of the position of the affected part AP.

According to the variation 2, the drive signal changes according to the position of the affected part AP. Thereby, the restriction on the position of the affected part AP in the treatment can be removed.

The variation 2 is particularly useful when the affected part AP cannot be fixed (for example, the affected part AP is a serious wound, or the treatment target is a small animal).

(6) Summary of the present embodiment

Hereinafter, the present embodiment is briefly described.

A first aspect of the present embodiment is

An ultrasonic therapy apparatus 1 comprising:

> a plurality of ultrasonic transducers 21;
> means for identifying a position of an affected part AP (for example, the processor 12 executing step S101);
> means for determining a focal point of ultrasonic waves radiated by the plurality of ultrasonic transducers based on the identified position of the affected part AP (for example, the processor 12 executing step S102); and
> a drive circuit 15 that generates a drive signal DRV that drives the plurality of ultrasonic transducers 21 at individual timings so that the ultrasonic waves are focused at the determined focal point.

According to the first aspect, since the ultrasonic wave USW is focused on the focal point FP determined based on the position of the affected part AP, the acoustic radiation pressure ARP generated at the focal point FP directly propagates to the affected part AP. The acoustic radiation pressure ARP is transmitted directly to the affected part AP without passing through a medium (for example, air or medicine). Therefore, there is no need to bring the ultrasonic radiation unit 20 into contact with the affected part AP, and it is not necessary to apply a medicine to the affected part AP. As a result, the risk of infection in treatment using ultrasonic can be reduced.

In a second aspect of the present embodiment,
the drive circuit 15 generates a drive signal DRV having a frequency.

According to the second aspect, since the acoustic radiation pressure ARP has a frequency (that is, vibration occurs at the focal point FP), the recovery of the affected part AP can be further promoted.

In a third aspect of the present embodiment,
the means for determining the frequency determines the frequency based on treatment target information regarding a treatment target, and
the drive circuit 15 generates a drive signal DRV having the determined frequency.

According to the third aspect, since the ultrasonic waves USW corresponding to the vibration frequency determined based on the treatment target information are focused, the acoustic radiation pressure ARP corresponding to the treatment target is directly propagated to the affected part AP. Therefore, the optimal acoustic radiation pressure ARP for the treatment target can be transmitted to the affected part AP.

In a fourth aspect of the present embodiment,
the apparatus comprises means for determining an ultrasonic parameter including at least one of a radiation time, an amplitude, and a modulation type of the ultrasonic wave radiated from each of the ultrasonic transducers 21 (for example, the processor 12 executing step S104); and
the drive circuit 15 generates a drive signal DRV according to the determined ultrasonic parameter.

According to the fourth aspect, at least one of the radiation time, amplitude, and modulation type of the ultrasonic wave is variable. Thereby, the recovery of the affected part AP can be further promoted.

In a fifth aspect of the present embodiment, the means for determining the ultrasonic parameter determines the ultrasonic parameter based on treatment target information regarding a treatment target.

According to the fifth aspect, since the ultrasonic waves USW corresponding to the ultrasonic parameter determined based on the treatment target information are focused, the acoustic radiation pressure ARP corresponding to the treatment target is directly propagated to the affected part AP. Therefore, the optimal acoustic radiation pressure ARP for the treatment target can be transmitted to the affected part AP.

In the sixth aspect of the present embodiment, the treatment target information is at least one of biological information of the treatment target, an attribute of the treatment target, and an attribute of the affected part AP.

According to the sixth aspect, the acoustic radiation pressure ARP corresponding to at least one of the biological information of the treatment target, the attribute of the treatment target, and the attribute of the affected part AP can be directly transmitted to the affected part AP.

In a seventh aspect of the present embodiment, the biological information includes at least one of a blood pressure, and a heart rate of the treatment target.

According to the seventh aspect, the acoustic radiation pressure ARP corresponding to at least one of the blood pressure, and the heart rate of the treatment target can be directly transmitted to the affected part AP.

In an eighth aspect of the present embodiment, the attribute of the affected part AP includes at least one of the area and the type of the affected part AP.

According to the eighth aspect, the acoustic radiation pressure ARP corresponding to at least one of the area and the type of the affected part AP can be directly transmitted to the affected part AP.

In a ninth aspect of the present embodiment, the determining means determines a position and a number of the focal point based on a shape of the affected part AP.

According to the ninth aspect, the acoustic radiation pressure ARP corresponding to the shape of the affected part AP can be directly transmitted to the affected part AP.

In a tenth aspect of the present embodiment, the means for identifying identifies the position of the affected part AP based on a user's instruction.

According to the tenth aspect, it is possible to generate the acoustic radiation pressure ARP at the focus FP arbitrarily set by the user.

In an eleventh aspect of the present embodiment, the means for identifying identifies the position of the affected part AP by analyzing an image.

According to the eleventh aspect, the acoustic radiation pressure ARP can be generated at the focal point FP recognized by the ultrasonic therapy apparatus 1.

(7) Other Variations

Other Variations will be described.

The ultrasonic therapy apparatus 1 is applied to, for example, the following devices.

- Wound treatment apparatus
- Infection treatment apparatus
- Beauty care apparatus
- Anti-aging care apparatus
- Skin care apparatus
- Hair care apparatus
- Animal treatment apparatus
- Animal care apparatus

For example, when applying the ultrasonic therapy apparatus 1 to a beauty care apparatus, by radiating ultrasonic waves to the skin, it is possible to produce collagen fibers (collagen) having a cosmetic effect.

For example, when the ultrasonic therapy apparatus 1 is applied to a hair care apparatus, the growth of the hair (that is, hair growth) can be promoted by radiating the ultrasonic waves to the head.

For example, in a case where the ultrasonic therapy apparatus 1 is applied to an animal treatment apparatus, the risk of infection can be reduced by radiating ultrasonic waves to an affected part of the animal, as in the present embodiment.

Although the embodiments of the present invention are described in detail above, the scope of the present invention is not limited to the above embodiments. Further, various modifications and changes can be made to the above embodiments without departing from the spirit of the present invention. In addition, the above embodiments and variations can be combined.

[Reference Signs List]

1: Ultrasonic therapy apparatus
10: Controller
11: Storage device
12: Processor
13: Input / output interface
15: Drive circuit
16: Operation unit
17: Display unit
20: Ultrasonic radiation unit
21: Ultrasonic transducer
22: Camera

**Claims**

1. An ultrasonic therapy apparatus, comprising:

    a plurality of ultrasonic transducers;
    a processor,
    identifying a position of an affected part;
    determining a focal point of ultrasonic waves radiated by the plurality of ultrasonic transducers based on the identified position of the affected part; and
    a drive circuit that generates a drive signal for driving the plurality of ultrasonic transducers at individual timings so that the ultrasonic waves are focused at the determined focal point.

2. The ultrasonic therapy apparatus according to claim 1, wherein the drive circuit generates a drive signal having a frequency.

3. The ultrasonic therapy apparatus according to claim 2, wherein the processor determines the frequency of the drive signal based on treatment target information related to a treatment target, and
   the drive circuit generates a drive signal having the determined frequency.

4. The ultrasonic therapy apparatus according to any one of claims 1 to 3, wherein the processor determines an ultrasonic parameter including at least one of a radiation time, an amplitude, and a modulation type of the ultrasonic wave radiated from each of the ultrasonic transducers, and wherein the drive circuit generates a drive signal according to the determined ultrasonic parameter.

5. The ultrasonic therapy apparatus according to claim 4, wherein the processor determines the ultrasonic parameter determines the ultrasonic parameter based on treatment target information.

6. The ultrasonic therapy apparatus according to claim 3 or 5, wherein the treatment target information is at least one of biological information of the treatment target, an attribute of the treatment target, and an attribute of the affected part.

7. The ultrasonic therapy apparatus according to claim 6, wherein the biological information includes at least one of a blood pressure, and a heart rate of the treatment target.

8. The ultrasonic therapy apparatus according to claim 6 or 7, wherein the attribute of the affected part includes at least one of an area, and a type of the affected part.

9. The ultrasonic therapy apparatus according to any one of claims 1 to 8, wherein the processor determines a position, and a number of the focal point based on a shape of the affected part.

10. The ultrasonic therapy apparatus according to any one of claims 1 to 9, the processor identifies the position of the affected part based on a user's instruction.

11. The ultrasonic therapy apparatus according to any one of claims 1 to 9, wherein the processor identifies the position of the affected part by analyzing an image.

12. The ultrasonic therapy apparatus according to any one of claims 1 to 11, wherein the ultrasonic therapy apparatus is a wound treatment apparatus, an infection treatment apparatus, a beauty care apparatus, an anti-aging care apparatus, a skin care apparatus, a hair care apparatus, an animal treatment apparatus, or an animal care apparatus.

EP 3 689 418 A1

[FIG. 1]

[FIG. 2]

1

```
                    16              17
              ┌──────────┐   ┌──────────┐
              │Operation │   │          │
              │  Unit    │   │Display Unit│
              └──────────┘   └──────────┘
    11              12              13
┌──────────────┐ ┌──────────┐ ┌──────────────┐
│              │ │          │ │Input / Output│
│Storage Device│ │ Processor│ │  Interface   │
└──────────────┘ └──────────┘ └──────────────┘

         15
    ┌──────────────┐
    │ Drive Circuit│
    └──────────────┘
10

20
    ┌──────────────┐   21    ┌──────────────┐
    │  Ultrasonic  │         │              │
    │  Transducer  │         │   Camera     │
    └──────────────┘         └──────────────┘
                              22
```

[FIG. 3]

[FIG. 4]

FP:(xfp,yfp,zfp)

FA

21c(n):(x(n),y(n),z(n))

r(n)

r(n+1)

21c(n+1):(x(n+1),y(n+1),z(n+1))

Z+ X+

Y+

Y−

X−  Z−

[FIG. 5]

[FIG. 6]

[FIG. 7]

[FIG. 8]

```
                    ┌─────────────────────────────┐
                    │            START            │
                    └─────────────────────────────┘
                                   │
                                   │                    S100
           ┌───────────────────────────────────────────┐
           │     Identification of Shape of Affected Part │
           └───────────────────────────────────────────┘
                                   │                    S101
           ┌───────────────────────────────────────────┐
           │     Identification of Position of Affected  │
           │                    Part                     │
           └───────────────────────────────────────────┘
                                   │                    S102
           ┌───────────────────────────────────────────┐
           │         Determination of Focal Point        │
           └───────────────────────────────────────────┘
                                   │                    S103
           ┌───────────────────────────────────────────┐
           │        Calculation of Phase Difference      │
           └───────────────────────────────────────────┘
                                   │                    S104
           ┌───────────────────────────────────────────┐
           │    Determination of Ultrasonic Parameter    │
           └───────────────────────────────────────────┘
                                   │                    S105
           ┌───────────────────────────────────────────┐
           │   Determination of Vibration Frequency      │
           │      of Acoustic Radiation Pressure         │
           └───────────────────────────────────────────┘
                                   │                    S106
           ┌───────────────────────────────────────────┐
           │         Generation of Drive Signal          │
           └───────────────────────────────────────────┘
                                   │                    S107
           ┌───────────────────────────────────────────┐
           │        Radiation of Ultrasonic Waves        │
           └───────────────────────────────────────────┘
                                   │
                                   ▼
                    ┌─────────────────────────────┐
                    │             END             │
                    └─────────────────────────────┘
```

[FIG. 9]

[FIG. 10]

FIG. 10A

IMG1          AP          IMG2

16

17

FIG. 10B

OBJ          AP

22

Camera

Image Data

10

Controller

Image Analysis

Identification of
Affected Part

[FIG. 11]

[FIG. 12]

[FIG. 13]

[FIG. 14]

Non-irradiated

Irradiated

Sparse Collagen Fibers

Dense Collagen Fibers

Irradiation Condition: 10 Hz, 90.6 Pa, 1 hour/day,
Irradiated for 3 consecutive days, Tissue was collected day 7

[FIG. 15]

[FIG. 16]

Non-irradiated    Side Surface    Irradiated

Granulation tissue

Spine Side

500μm    500μm

Irradiation Condition: 10 Hz, 90.6 Pa, 1 hour/day,
Irradiated for 3 consecutive days, Tissue was collected day 14

[FIG. 17]

Non-irradiated     Irradiated

[FIG. 18]

[FIG. 19]

[FIG. 20]

[FIG. 21]

[FIG. 22]

[FIG. 23]

[FIG. 24]

[FIG. 25]

[FIG. 26]

[FIG. 27]

| Prameter Determination Table | | | | | | | | | |
|---|---|---|---|---|---|---|---|---|---|
| Inependent Variable | | | | | Dependent Variable | | | | |
| Treatment Target Attribute | Biological Information | | Affected Part Attribute | | Vibration Frequency | Ultrasonic Paramter | | | |
| | Blood Pressure | Heart Rate | Area | Type | | Radiation Time | Amplitude | Modulation Type | |
| HUMAN | 100 | 40 | 60 | Wound | 1 | 10 | 5 | AM | |
| HUMAN | 90 | 50 | 30 | Burn | 10 | 5 | 10 | FM | |
| . | . | . | . | . | . | . | . | . | |
| MOUSE | 60 | 400 | 10 | Laceration | 100 | 1 | 2 | FM | |
| . | . | . | . | . | . | . | . | . | |

## INTERNATIONAL SEARCH REPORT

| International application No. |
| --- |
| PCT/JP2018/034447 |

A.  CLASSIFICATION OF SUBJECT MATTER
Int.Cl. A61N7/00(2006.01)i

According to International Patent Classification (IPC) or to both national classification and IPC

B.  FIELDS SEARCHED

Minimum documentation searched (classification system followed by classification symbols)
Int.Cl. A61N7/00

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | WO 2014/135987 A2 (INSIGHTEC, LTD.) 12 September 2014, paragraphs [0003]-[0006], [0008]-[0013], [0023], [0027]-[0028], fig. 1-2, 4 & JP 2016-508808 A & US 2016/0008633 A1 & CN 105339045 A | 1-12 |
| X | JP 2000-237199 A (TOSHIBA CORP.) 05 September 2000, paragraphs [0015]-[0035], fig. 1-2 & US 2003/0149380 A1, paragraphs [0057]-[0083], fig. 1-2 | 1-12 |
| A | WO 2006/123414 A1 (TECHNO LINK CORPORATION) 23 November 2006, entire text, all drawings (Family: none) | 1-12 |
| A | US 2011/0319793 A1 (HYNYNEN, Kullervo Henrik) 29 December 2011, entire text, all drawings & WO 2012/006053 A1 | 1-12 |

☐ Further documents are listed in the continuation of Box C.  ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 24 October 2018 (24.10.2018) | 06 November 2018 (06.11.2018) |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007521053 A